# EUROPEAN PATENT APPLICATION

(11) **EP 4 722 607 A1**
(43) Date of publication of application: **08.04.2026**
(21) Application number: 24815584.8
(22) Date of filing: 30.05.2024
(51) Int. Cl.: F24F 7/06, B01L 1/00

(54) **CLEAN ROOM SYSTEM AND VEHICLE MOUNTED WITH CLEAN ROOM SYSTEM**

(30) Priority: 01.06.2023 JP 2023091146
(71) Applicant: Gaudi Clinical Co., Ltd., Tokyo 113-0033 (JP)
(72) Inventor: TANAKA, Masanori, Tokyo 113-0033 (JP)
(74) Representative: ABG Intellectual Property Law, S.L.
(86) International application number: PCT/JP2024/019928
(87) International publication number: WO 2024/248104

(57) **Abstract**

A cleanroom system capable of reducing the installation or maintenance costs is realized. A cleanroom system 10 includes a cleanroom 20 having airtightness, an air supply unit 30 that supplies air from an exterior 90 to an interior 22 of the cleanroom, and a safety cabinet 40. The safety cabinet 40 includes a workspace 42, a workspace air supply path that supplies air from the interior 22 to the workspace 42, a workspace exhaust path that exhausts air from the workspace 42 to the exterior 90, an exhaust fan 60 that is provided in the workspace exhaust path and drives exhaust, and a filter 62 that is provided in the workspace exhaust path and purifies the exhaust. The exhaust fan 60 drives total exhaust from the interior 22 to the exterior 90 and maintains the interior 22 at negative pressure.

## Description

### TECHNICAL FIELD

The present invention relates to a cleanroom system and a vehicle mounted with the cleanroom system.

### BACKGROUND ART

In a negative-pressure cleanroom in which an internal pressure is maintained lower than an external pressure, air flows from the exterior to the interior of the cleanroom in a communicating portion with the exterior. Therefore, in a negative-pressure cleanroom, it is possible to reduce or prevent leakage of air containing airborne fine particles such as infectious substances present inside the cleanroom to the exterior of the cleanroom.

A cleanroom may be provided with a device such as a safety cabinet that includes a workspace for performing operations on work objects such as infectious substances. In many cases, the safety cabinet controls the supply and exhaust of air from the workspace by itself.

Non-Patent Literature 1 is Japanese Industrial Standard JIS for safety cabinets and similar devices. The device is referred to as a biohazard control cabinet, and is classified into Class I, Class II, and Class III according to its basic structure. Class I and Class II are open-type devices with an opening (front opening) in the front wall of the workspace, while Class III is a closed-type device without any opening.

Class I devices are generally referred to as draft chambers, which supply air drawn from a front opening to a workspace without purification, and exhaust air from the workspace after purification by a filter. Class II devices are commonly referred to as safety cabinets. Among them, in the type equipped with a circulation path, the air drawn from the front opening is taken into the circulation path. The circulating air is purified by the filter, supplied to the workspace, and exhausted from the workspace, repeating this process. In this process, a portion of the air is purified by a filter and then exhausted to the exterior. In the type without a circulation path, air separately drawn from the exterior is purified by a filter and supplied to the workspace, while the air supplied to the workspace and the air supplied from the front opening are collectively purified by a filter and exhausted to the exterior. Class III devices are generally called isolators, in which air drawn from the exterior is purified by a filter and supplied to a workspace, and the exhaust air from the workspace is purified by a filter and exhausted to the exterior. Although this standard is labeled as "bio", it is not limited to applications in the biological field, and it has been formulated with consideration for other uses, such as handling other substances such as volatile substances.

Patent Literature 1 below describes a negative-pressure cleanroom formed by combining partitions. This cleanroom is used for biological applications, and a safety cabinet is installed therein. In this cleanroom, the air supply/exhaust systems of the cleanroom itself and the safety cabinet are integrated. Specifically, a supply fan supplies air to the cleanroom, and an exhaust fan exhausts air from the cleanroom to the exterior. A local exhaust fan that exhausts air from the safety cabinet to the exterior of the cleanroom is also separately provided. These fans are controlled by a control unit and keep the interior of the cleanroom at negative pressure.

Patent Literature 2 below is a literature related to a slightly positive-pressure open-type cleanroom (referred to as a cleanbooth in this literature) that employs an airflow barrier system and is equipped with a safety cabinet and the like. The wall of the cleanroom is provided with a constantly open entrance without a door, and the ceiling is provided with a supply fan. Air is supplied into the cleanroom by a supply fan. In the safety cabinet, a fan provided in the safety cabinet supplies air inside the cleanroom, causes the air to flow into a workspace, and then exhausts the air from the safety cabinet into the cleanroom. The air inside the cleanroom flows in one direction toward the entrance/exit and is exhausted to the exterior of the cleanroom.

Patent Literature 3 below describes a technique in which cells collected from a patient are processed and then transplanted to a patient. Cells collected in academia are processed in a cell culture processing facility (referred to as CPF in the literature), returned to academia, and used for transplantation into patients in academia. Cells to be transplanted are treated as pharmaceuticals, and manufacturers are required to perform a series of process controls under CGMP grade in accordance with US laws and regulations (paragraphs 0012 to 0018, etc.). Therefore, this literature proposes that a facility that satisfies the CGMP grade is provided near a place where a patient receives treatment, and necessary management or the like is performed (paragraphs 0019, 0089 to 0118, etc.).

In the case of performing regenerative medicine, it is necessary to comply with the laws and regulations of the country or region in which it is practiced. In Japan, the term "regenerative medical products, etc." is defined in the law of Non-Patent Literature 2 as follows: "The term refers to the following items (excluding quasi-drugs and cosmetics) as specified by government ordinance: (i) Items intended for use in medical or veterinary treatment that involve cultured or otherwise processed human or animal cells, for the purpose of: (a) Reconstructing, repairing, or forming the structure or function of the human or animal body; or (b) Treating or preventing diseases in humans or animals; and (ii) Items intended for use in the treatment of diseases in humans or animals, which contain genes introduced into human or animal cells and are expressed within the body". In addition, the law of Non-Patent Literature 3 defines, for example, provisions related to the safety assurance of regenerative medicine, including regenerative medical products.

### CITATION LIST

### PATENT LITERATURE

Patent Literature 1: JP 2005-326093 A
Patent Literature 2: JP 2019-100594 A
Patent Literature 3: US Patent Publication No. 2003-0175242A1

### NON-PATENT LITERATURE

Non-Patent Literature 1: JIS K3800 Class II Biohazard Control Cabinet, revised on July 20, 2021
Non-Patent Literature 2: "Act on Ensuring Quality, Effectiveness, and Safety of Pharmaceuticals, Medical Devices, etc." (Japan)
Non-Patent Literature 3: "Act on Safety Assurance of Regenerative Medicine, etc." (Japan)

### SUMMARY OF INVENTION

### PROBLEMS TO BE SOLVED BY THE INVENTION

There is a need to simply configure a negative-pressure cleanroom. However, the cleanroom of Patent Literature 1 described above is provided with an exhaust fan that exhausts air from the interior of the cleanroom to the exterior and a local exhaust fan that exhausts air from the interior of the safety cabinet to the exterior of the cleanroom. Thus, the main exhaust system of the cleanroom and the local exhaust system via the safety cabinet are integrally controlled, so that the exhaust structure has redundancy. Patent Literature 2 described above describes a simple exhaust structure, but this is merely a configuration specific to a positive-pressure cleanroom.

An object of the present invention is to realize a negative-pressure cleanroom system which reduces the manufacturing or maintenance costs.

In addition, in order to smoothly carry out regenerative medicine, there is a need to provide a cleanroom for handling cells in or near a hospital or the like where a patient receives treatment. However, in Patent Literature 3 described above, the costs associated with the installation or maintenance of the facility are not considered.

Another object of the present invention is to facilitate installation or maintenance of a cleanroom in or near a hospital or the like that performs regenerative medicine.

### MEANS FOR SOLVING THE PROBLEMS

A cleanroom system according to the present invention includes: a cleanroom having airtightness; a room air supply path that supplies air from an exterior to an interior of the cleanroom; and a hazard control cabinet installed inside the cleanroom. The hazard control cabinet including: a workspace; a workspace air supply path that supplies air from the interior of the cleanroom to the workspace; a circulation path that takes in air from the workspace and supplies the air to the workspace again; a workspace exhaust path that exhausts air from the workspace to the exterior of the cleanroom; an exhaust fan that drives circulation in the circulation path and exhaust in the exhaust path; and a filter that is provided in the workspace exhaust path and purifies the exhaust. The exhaust fan drives circulation in the circulation path and exhaust in the exhaust path and total exhaust from the interior of the cleanroom to the exterior, and maintains the interior of the cleanroom at negative pressure. The cleanroom system may include a circulation path that takes in air from the workspace and supplies the air to the workspace again. The exhaust fan may drive circulation in the circulation path and exhaust in the exhaust path.

The workspace is a space in which work is performed on a work object by an operator or equipment. The hazard control cabinet is a device that includes a workspace in which the supply and exhaust of air are controlled, and performs hazard control due to fine particles generated in the workspace. Hazard control refers to control for reducing or eliminating risks to operators or the surrounding environment. The hazard control cabinet includes, but is not limited to, the biohazard control cabinet having the basic structures of classes I, II, and III described in Non-Patent Literature 1. The hazard control cabinet includes a device that does not satisfy conditions such as numerical values, configurations, and materials defined in Non-Patent Literature 1, but can implement a level of hazard control required by a user as a whole.

Maintaining the interior of the cleanroom at negative pressure means either or both of the conditions: the air pressure inside the cleanroom is controlled to be lower than the external air pressure on a time-averaged basis; and the airflow is directed from the exterior to the interior of the cleanroom on a time-averaged basis. Since the air pressure fluctuates temporally and spatially due to the operation of fans, the turbulence in the flow, and the like, a smoothed state of such fluctuations can be evaluated by taking a time average. The period for taking the time average may depend on the response speed of the pressure sensor, and may be, for example, about 60 seconds, 30 seconds, or 10 seconds. The direction of airflow can be confirmed by a smoke test. The pressure difference between inside and outside can be set as appropriate, and specific values such as 30 Pa, 20 Pa, 10 Pa, 5 Pa, 3 Pa, or 1 Pa can be exemplified. It is conceivable that the pressure difference is set according to a level at which leakage of fine particles in the air from the interior of the cleanroom to the exterior is to be reduced or prevented. Furthermore, it is also conceivable that the pressure difference is set in consideration of the ease of the opening/closing operation of the entrance/exit door, the suppression of wind noise, the power consumption of fans, or the air permeability of filters. When the pressure difference is set small, the air pressure inside the cleanroom may be higher than the outside air pressure for a short time (positive pressure is created). However, if the amount of air leakage to the exterior occurs as a result of the positive pressure is small, and the cleanroom system can maintain a level sufficient for hazard control, such short-time positive pressure may be tolerated.

In the cleanroom system, the hazard control cabinet includes a workspace air supply path, a workspace exhaust path, an exhaust fan, and a filter, but these components are inseparable components closely related to the supply and exhaust of air in the cleanroom itself. In this respect, the cleanroom and the hazard control cabinet can be said to be integrally configured. Therefore, some or all of these components may be handled, in product catalogs or the like, as components provided in a cleanroom or separately provided components, rather than as components belonging to the hazard control cabinet. Even in this case, if these components are necessary elements for realizing a biohazard control cabinet, these components constitute components of the biohazard control cabinet, and can be said to satisfy the requirements of the present invention.

According to an aspect of the present invention, a bypass path is provided that communicates with the workspace exhaust path from the interior of the cleanroom without passing through the workspace, and the exhaust fan also drives air flowing through the bypass path. In another aspect, the bypass path is not provided, and only the air having passed through the workspace is exhausted.

In one aspect of the present invention, the hazard control cabinet is a safety cabinet, and the workspace is a space in which work is performed on biological objects. The safety cabinet includes, but is not limited to, those satisfying the Class II biohazard control cabinet in Non-Patent Literature 1. Even if conditions such as numerical values, configurations, and materials defined in Non-Patent Literature 1 are not satisfied, it is acceptable as long as a level of biohazard control required by a user as a whole can be implemented.

In one aspect of the present invention, the room air supply path is a natural air supply path that supplies air using negative pressure inside the cleanroom formed by the exhaust fan as a driving force.

In one aspect of the present invention, the workspace is a space in which work is performed on regenerative medical products manufactured in a cell culture processing facility, and the cleanroom system is installed near treatment locations where the regenerative medical products are used.

The regenerative medical products include those defined as regenerative medical products in Non-Patent Literature 2, but are not limited thereto. The regenerative medical products also include general cell-processed products obtained by culturing or other processing animal cells. In addition, the regenerative medical products include products classified as pharmaceutical products, quasi-drugs, cosmetics, veterinary pharmaceuticals, and the like by laws and regulations. Similarly, the treatment location refers not only to a place for treating patients, but also includes a place for performing procedures on persons or animals not treated as medical care under laws and regulations.

The regenerative medical products include those provided free of charge as well as those provided for a fee.

In an aspect of the present invention, the treatment locations are distributed in two or more buildings, and the cleanroom system is installed at a position near the treatment locations in at least two or more buildings.

A vehicle mounted with a cleanroom system according to the present invention includes the cleanroom system and a vehicle on which the cleanroom is mounted, the workspace is a space in which work is performed on regenerative medical products manufactured in a cell culture processing facility, and the vehicle is capable of moving near a treatment location where regenerative medical products are used.

### ADVANTAGEOUS EFFECTS OF THE INVENTION

A cleanroom system capable of reducing the manufacturing or maintenance costs is realized.

### BRIEF DESCRIPTION OF DRAWINGS

FIG. 1 is a perspective view of a cleanroom system according to a first embodiment.
FIG. 2 is a plan view of the cleanroom system according to the first embodiment.
FIG. 3 is a side view of the cleanroom system according to the first embodiment.
FIG. 4 is a diagram illustrating a control mode of the cleanroom system according to the first embodiment.
FIG. 5 is a side view of a cleanroom system according to a second embodiment.
FIG. 6 is a side view of a cleanroom system according to a third embodiment.
FIG. 7 is a side view of a cleanroom system according to a fourth embodiment.
FIG. 8 is a side view of a cleanroom system according to a fifth embodiment.
FIG. 9 is a diagram for describing a usage example of a cleanroom system according to a sixth embodiment.

### DESCRIPTION OF EMBODIMENTS

Hereinafter, embodiments of the present invention will be described with reference to the drawings. It should be noted that each embodiment is illustrative, and the present invention can be implemented in various other forms. In addition, it should be noted that each figure is a schematic diagram simply showing the features of the embodiment, and the structure and scale are not necessarily accurate.

### (1) First Embodiment

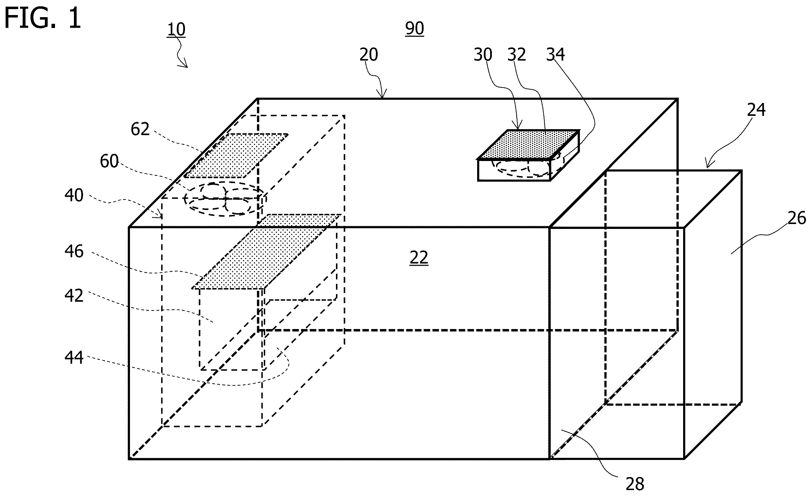

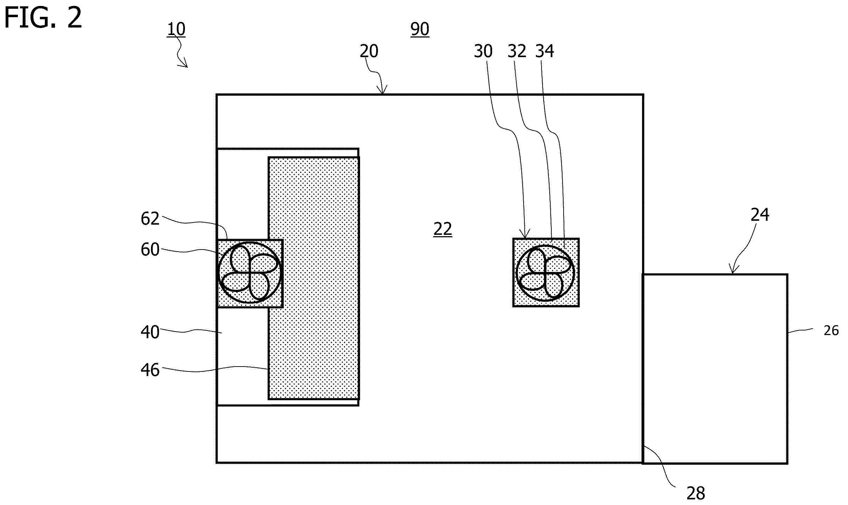

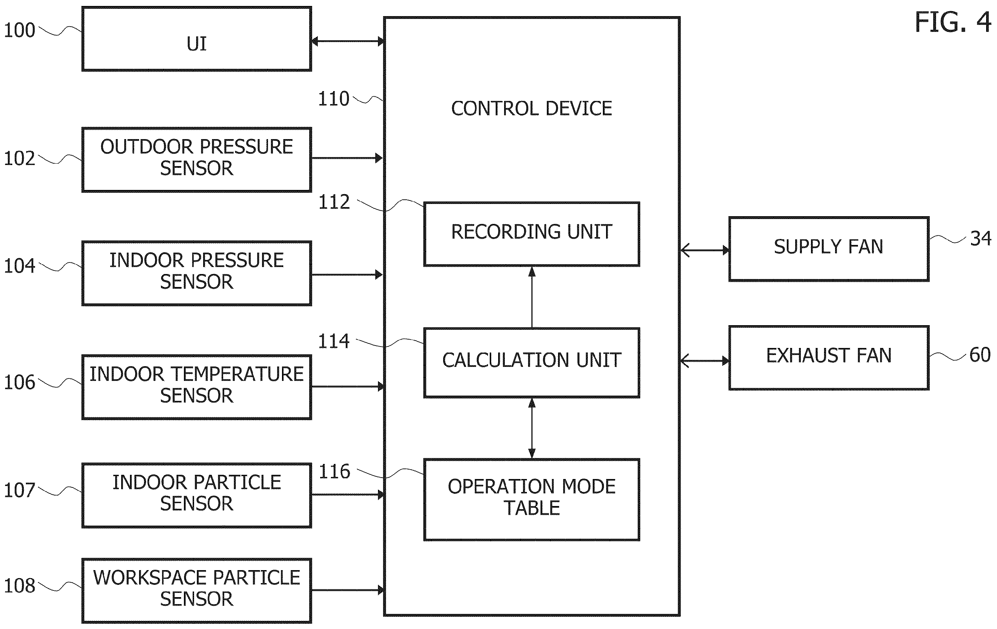

FIGS. 1, 2, and 3 are a perspective view, a plan view, and a side view of a cleanroom system 10 according to the first embodiment, respectively. FIG. 4 is a block diagram illustrating a control mode of the cleanroom system 10 according to the first embodiment.

First, an overall configuration of a cleanroom system 10 will be described with reference to FIGS. 1 to 3. The cleanroom system 10 is a system formed by a cleanroom 20 in which cleanliness is controlled, devices associated therewith, and the like.

The cleanroom 20 is a closed structure that includes a floor, walls, and a ceiling and keeps the space of the interior 22 in an airtight state. Airtightness refers to a state in which the flow of air between an interior 22 and an exterior 90 is reduced or blocked, and airtightness performance refers to the property of keeping a space in an airtight state. Since the cleanroom 20 has airtightness, cleanliness in the interior 22 of the cleanroom 20 can be controlled. The cleanliness is quantified by, for example, the amount of fine particles in the air. The floor, walls, and ceiling of the cleanroom 20 are formed by, for example, tightly combining panel members manufactured in a quality-controlled factory. Some or all of the walls, floors, and ceiling of the cleanroom 20 can also be formed using the structure of an existing building. When the cleanroom 20 is installed in a building, the exterior 90 of the cleanroom 20 may be a general indoor space where cleanliness is not controlled, or may be another cleanroom where cleanliness is controlled. Further, it is also possible to install the cleanroom 20 outdoors in a form in which the outer surface thereof is exposed to the outdoors. Note that, in FIGS. 1 to 3, the cleanroom 20 is illustrated on the assumption that it is about 3 meters in each of the two horizontal directions and about 2 meters in height. However, the size can be set in various ways, and may be smaller (for example, about 2 meters in one or both of the two horizontal directions) or larger (for example, about 4, 6, or 10 meters in one or both horizontal directions, or about 3 or 4 meters in height).

The cleanroom system 10 according to the first embodiment includes a buffer room 24 adjacent to the cleanroom 20 in addition to the cleanroom 20. The buffer room 24 is a room that serves as a buffer space between the interior 22 of the cleanroom 20 and the exterior 90, when an operator enters or leaves or when a work object is brought in or out. The buffer room 24 includes an outer door 26 and an inner door 28. The outer door 26 is used for entry and exit between the exterior 90 and the buffer room 24, and the inner door 28 is used for entry and exit between the buffer room 24 and the interior 22 of the cleanroom 20. The outer door 26 and the inner door 28 are controlled so as not to be opened simultaneously, and direct ventilation between the interior 22 of the cleanroom 20 and the exterior 90 is prevented. In addition, by providing the buffer room 24, it is possible to reduce a change in air pressure in the interior 22 of the cleanroom 20 during entry and exit and to suppress air turbulence.

The cleanroom 20 is provided with an air supply unit 30 and a safety cabinet 40 as devices constituting the cleanroom system 10.

The air supply unit 30 is a device that controls supply of air to the cleanroom 20. The air supply unit 30 is installed in a flow path (referred to as a "room air supply path") for supplying air from the exterior 90 to the interior 22 of the cleanroom 20. In the cleanroom system 10, the room air supply path is provided at a position on the ceiling of the cleanroom 20 relatively close to the buffer room 24. The air supply unit 30 is a unitized device in which the HEPA filter 32 and the supply fan 34 are combined, and is detachably attached to the room air supply path. The HEPA filter 32 is a filter that removes fine particles in the air according to a high-efficiency particulate air (HEPA) standard. The HEPA filter 32 is detachably attached to the supply fan 34. Therefore, the HEPA filter 32 can be easily cleaned and replaced by removing the air supply unit 30 and further removing the HEPA filter 32.

The supply fan 34 is a device including a fan for supplying air. The term "fan" broadly refers to a device that drives air, and is intended to include those of various structures, such as a propeller fan, a sirocco fan, a centrifugal fan, or a combined fan thereof. The supply fan 34 operates by receiving power from a power line (not shown), and drives air from the exterior 90 to the interior 22 of the cleanroom 20. At this time, since the air flows through the HEPA filter 32, fine particles contained in the air from the exterior 90 are removed.

The safety cabinet 40 is an open-type hazard control cabinet having a front opening 44. The safety cabinet 40 is provided near a wall surface of the interior 22 of the cleanroom 20, far from the buffer room 24. The safety cabinet 40 includes a workspace 42, the front opening 44, a HEPA filter 46, flow paths 50a to 50f, an exhaust fan 60, and a HEPA filter 62.

The workspace 42 is a space provided for performing work on a work object, and may be referred to as a work hood, a work chamber, or the like. The workspace 42 is provided within the safety cabinet 40 in a substantially rectangular parallelepiped shape. The HEPA filter 46 is provided on an upper surface of the workspace 42. A wall of a front surface (in the figure, a side close to the center of the room, i.e., a side on which the operator is positioned) of the workspace 42 is formed of a transparent glass plate, but a lower part thereof is the front opening 44 without a resin plate. A bottom surface of the workspace 42 or a periphery thereof is provided with a hole communicating with the flow path 50a described later.

The front opening 44 is a portion communicating with the interior 22 of the cleanroom 20, and is used not only for an operator to insert and remove an arm and a work object, but also as a flow path for supplying air from the interior 22 of the cleanroom 20 to the device.

The HEPA filter 46 is installed on the upper surface of the workspace 42. The HEPA filter 46 is a filter that purifies air supplied to the workspace 42, and air flows from the upper surface to the lower surface thereof. The air that has passed through the HEPA filter 46 is purified to a level at which an operation called an aseptic operation can be performed in the workspace 42.

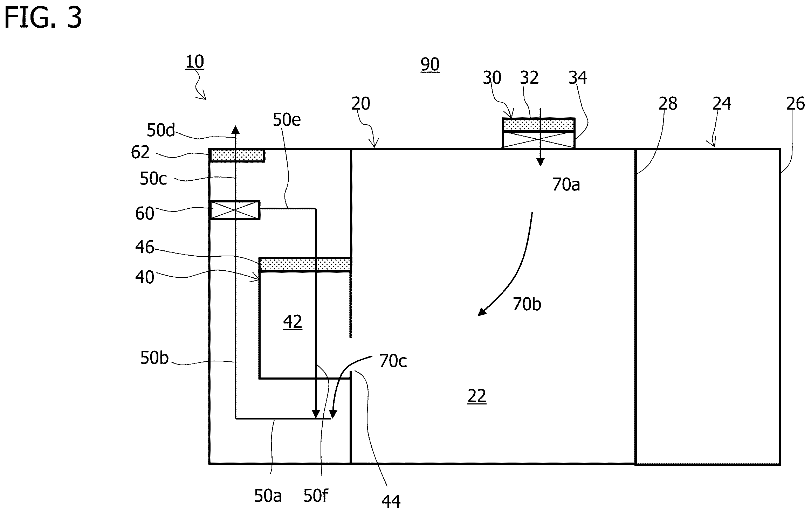

As illustrated in FIG. 3, the interconnected air flow paths 50a to 50f are provided around the workspace 42. The flow path 50a is provided below the workspace 42 and communicates with a hole provided in the bottom surface of the workspace 42. The flow path 50a extends substantially horizontally in the front-rear direction of the safety cabinet 40. The flow path 50b is a flow path that is provided behind the workspace 42 and extends in the up-down direction (vertical direction). The lower end of the flow path 50b communicates with the rear end of the flow path 50a. The flow path 50c is a flow path located on an extension line of the flow path 50b, and extends in the up-down direction. The lower end of the flow path 50c communicates with the upper end of the flow path 50b via the exhaust fan 60. The flow path 50d is a flow path located on an extension line of the flow path 50c, and extends substantially in the up-down direction. The lower end of the flow path 50d communicates with the upper end of the flow path 50c via the HEPA filter 62, and the upper end of the flow path 50d communicates with the exterior 90 of the cleanroom 20.

The flow path 50e is a flow path located above the workspace 42, and extends substantially horizontally in the front-rear direction of the safety cabinet 40. The rear end of the flow path 50e communicates with the upper end of the flow path 50b via the exhaust fan 60, and the front end of the flow path 50e is connected to the upper surface of the HEPA filter 46. The flow path 50f is an air flow path in the workspace 42, and flows substantially uniformly downward through the workspace 42. The upper end of the flow path 50f is connected to the lower surface of the HEPA filter 46 and is connected to the front end of the flow path 50e via the HEPA filter 46. The lower end of the flow path 50f communicates with the flow path 50a via a hole provided in the bottom surface of the workspace 42. A lower front part of the flow path 50f communicates with the interior 22 of the cleanroom 20 (and the exterior of the safety cabinet 40) via the front opening 44. Some or all of the flow paths 50a to 50e can be formed using, for example, a tubular duct, and can also be formed using a plate-like member. In the present embodiment, the flow paths 50a to 50e are attached to the frame constituting the safety cabinet 40, and the peripheries thereof are covered with the cover of the safety cabinet 40, and are in a state of being formed integrally with the safety cabinet 40.

The exhaust fan 60 is provided at a portion where the flow paths 50b, 50c, and 50e communicate with each other. The exhaust fan 60 includes a fan that drives air and is operated by electric power. The exhaust fan 60 sucks air from the flow path 50b, distributes the air to the flow path 50c and the flow path 50e, and discharges the air. The distribution ratio may be fixed, or may be variably controlled using, for example, an electrically-operated movable valve.

The HEPA filter 62 is detachably provided between the flow path 50c and the flow path 50d. The HEPA filter 62 is a filter that purifies the exhaust of air from the interior 22 of the cleanroom 20 to the exterior 90 to a level at which hazards can be prevented or reduced.

Typically, one HEPA filter 62 is provided at one location in the flow paths 50c and 50d, but it may be provided at a plurality of locations, or a plurality of HEPA filters may be provided at one location.

Next, the operation of the cleanroom system 10 will be described. In the cleanroom system 10, an operator enters the interior 22 of the cleanroom 20 from the exterior 90 via the buffer room 24. At this time, the operator first opens the outer door 26, enters the buffer room 24, and closes the outer door 26. When the buffer room 24 is used as a changing room, the operator puts on predetermined clothing or the like. Subsequently, the operator opens the inner door 28, enters the interior 22 of the cleanroom 20, and closes the inner door 28. The outer door 26 and the inner door 28 are controlled by interlock, and are configured such that both cannot be opened simultaneously.

In the interior 22 of the cleanroom 20, the operator is positioned in front of the safety cabinet 40 in a standing state or a seated state. Then, the operator operates the work object and the like while inserting and removing the work object and the arm into and from the workspace 42 through the front opening 44.

In the cleanroom 20, air 70a is supplied from the exterior 90 to the interior 22 through the air supply unit 30 provided in the room air supply path. The supply fan 34 drives the supply of air. Air 70a flows into the interior 22 of the cleanroom 20 after purification by the HEPA filter 32.

In the interior 22 of the cleanroom 20, air 70b flows from the air supply unit 30 toward the front opening 44 of the safety cabinet 40. Air 70b is controlled such that the turbulence is relatively small, and flows approximately in one direction. Then, air 70c is taken into the safety cabinet 40 through the front opening 44 and enters the flow path 50a.

Within the safety cabinet 40, air is driven by the exhaust fan 60 (of course, indirectly, the air supplied by the supply fan 34 also drives the air). The functions of the exhaust fan 60 can be classified into the following three categories. The first function is to supply air from the interior 22 of the cleanroom 20 to the workspace 42. The second function is to circulate air inside the safety cabinet 40. The third function is to exhaust air to the exterior 90. Each function will be described in detail below.

In the first air supply, air is supplied from the interior 22 to the workspace 42 via the front opening 44, the lower front part of the workspace 42 (also the lower front part of the flow path 50f), the flow path 50a, the flow path 50b, and the flow path 50e. This flow path supplies air from the interior 22 of the cleanroom 20 to the workspace 42, and is referred to as a "workspace air supply path". The exhaust fan 60 is provided in the middle of the workspace air supply path and drives the supply of air to the workspace 42. The HEPA filter 46 is provided in the workspace air supply path to purify the air supplied to the workspace 42.

In the second circulation, air flows through a circulation path consisting of the flow path 50e, the flow path 50f (workspace 42), the flow path 50a, and the flow path 50b. The exhaust fan 60 is provided in the circulation path and drives circulating air. The HEPA filter 46 provided in the circulation path purifies the air passing through it.

In this circulation path, the amount of air flowing into the flow path 50a from the hole provided in the bottom surface of the workspace 42 is larger than the amount of air supplied into the workspace 42 through the HEPA filter 46. This is because the exhaust fan 60 continuously exhausts a portion of the circulating air. As a result, the workspace 42 becomes deficient in air and negative pressure is created, and thus air is continuously sucked through the front opening 44 in the lower front part of the workspace 42. The continuous suction flow formed in the front opening 44 reduces or prevents fine particles flying inside the workspace 42 from leaking into the interior 22 of the cleanroom 20.

The third exhaust function can be considered from two perspectives. One is the perspective of exhausting air from the workspace 42 to the exterior 90. From this perspective, air flows from the workspace 42 through the flow paths 50a, 50b, 50c, and 50d to the exterior 90 via an exhaust path (referred to as a "workspace exhaust path"). The other is the perspective of exhausting air from the interior 22 of the cleanroom 20 to the exterior 90. From this perspective, air flows from the interior 22 through the front opening 44, the lower front part of the workspace 42 (the lower front part of the flow path 50f), and the flow paths 50a, 50b, 50c, and 50d to the exterior 90 via an exhaust path (referred to as a "room exhaust path"). The flow paths (referred to as "common exhaust paths") shared by the workspace exhaust path and the room exhaust path are the flow paths 50a, 50b, 50c, and 50d. The exhaust fan 60 and the HEPA filter 62 are both provided in the common exhaust path, and perform the driving and purification of the exhaust, respectively.

The exhaust fan 60 exhausts air by discharging a portion of the air sucked from the flow path 50b to the flow path 50c. The air flowing through the flow path 50b includes the air that has passed through the workspace 42 and the air that has entered from the front opening 44 but has not yet passed through the workspace 42. The latter air can be said to bypass the workspace 42 and be directly exhausted to the exterior 90 by merging into the workspace exhaust path via a "bypass path" consisting of the front opening 44 and the lower front part of the workspace 42, originating from the interior 22 of the cleanroom 20. The combination of the bypass path and the common exhaust path constitutes the room exhaust path. The exhaust fan 60 drives the flow in the bypass path.

The HEPA filter 62 purifies the air flowing from the flow path 50c to reduce fine particles in the air to a reference value or less, and then allows the air to flow into the flow path 50d. Accordingly, the leakage to the exterior 90, of fine particles that have entered the workspace 42 and are subject to hazard control is reduced or prevented (for example, the fine particles include infectious microorganisms, viruses, and the like, and encompass not only the substances themselves but also those in aerosolized form).

The exhaust performed to the exterior 90 via the exhaust fan 60 is the total exhaust from the interior 22 of the cleanroom 20 to the exterior 90. Here, "total exhaust" refers to the entire controlled exhaust volume exhausted from the cleanroom 20 during operation of the cleanroom system 10, and excludes air leaking from minute gaps in the walls of the cleanroom 20, air flowing backward turbulently from the air supply unit 30, air leaking from the buffer room 24 during entry and exit of operators, and the like. In the cleanroom system 10, since the exhaust fan 60 drives the total exhaust, the exhaust structure is simple, and the manufacturing or maintenance costs can be reduced. In addition, in the cleanroom system 10, there is a possibility that the manufacturing or maintenance costs can be reduced by reducing the installation locations or the number of HEPA filters 62 installed in the common exhaust path.

In the cleanroom system 10, the interior 22 of the cleanroom 20 is maintained at negative pressure. In the negative-pressure cleanroom 20 in which the interior 22 is maintained at negative pressure, it is possible to reduce or prevent the leakage of air from the walls of the cleanroom 20, the leakage of air from the air supply unit 30, and the leakage of air from the buffer room 24 during entry and exit. Therefore, it is possible to prevent or reduce the leakage of fine particles generated during work in the workspace 42 to the exterior 90.

The exhaust fan 60 is operated with power capable of maintaining the cleanroom 20 at negative pressure. The power required for the operation of the exhaust fan 60 also depends on the magnitude of the power of the supply fan 34. Therefore, in the cleanroom system 10, the exhaust fan 60 and the supply fan 34 are controlled while monitoring various states.

Here, an example of the control of the exhaust fan 60 and the supply fan 34 in the cleanroom system 10 will be described with reference to FIG. 4.

The UI 100 is a user interface including an input unit and a display unit formed by a touch panel display or the like. The UI 100 may be installed near the entrance of the cleanroom 20, or may be mounted on a terminal that can be carried by an operator, for example. The display unit of the UI 100 displays an operating status and the like of the cleanroom system 10, and the input unit receives various inputs from the user. Examples of the inputs include settings for switching the operating power between when the workspace 42 is in use and when it is not in use and settings for cleanliness, in addition to instructions to start or stop the cleanroom system 10.

An outdoor pressure sensor 102 and an indoor pressure sensor 104 are pressure sensors installed in the exterior 90 and the interior 22 of the cleanroom 20, respectively. An indoor temperature sensor 106 is a temperature sensor installed in the interior 22 of the cleanroom 20. An indoor particle sensor 107 and a workspace particle sensor 108 are installed in the interior 22 of the cleanroom 20 and the workspace 42, respectively, and measure airborne fine particles in the installed space. These various sensors perform measurements in real time.

The control device 110 is a device that operates by controlling computer hardware including a central processing unit, a memory, and the like using embedded software. In the control device 110, a recording unit 112, a calculation unit 114, and an operation mode table 116 are constructed under the control of the embedded software. The recording unit 112 records the operating status of the cleanroom system 10. The data to be recorded includes sensing data transmitted from various sensors to the control device 110, operational data transmitted from the supply fan 34 and the exhaust fan 60 to the control device 110, and the like. In addition, the recording unit 112 also records instruction data and setting data transmitted from the UI 100, control status data in the control device 110, and the like. The calculation unit 114 switches the operation modes of the supply fan 34 and the exhaust fan 60 in accordance with instruction data transmitted from the UI 100. Then, the calculation unit 114 refers to the operation mode table 116 and instructs the supply fan 34 and the exhaust fan 60 to operate in the corresponding operation mode. In addition, the calculation unit 114 monitors sensing data transmitted from various sensors to the control device 110, and performs feedback control to finely adjust the power of the supply fan 34 and the exhaust fan 60.

The operation mode table 116 is data in which the power of the supply fan 34 and the exhaust fan 60 is set for various operation modes. The operation mode is a mode in which the supply fan 34 and the exhaust fan 60 are operated. The operation mode includes a stop mode, a normal operation mode, a high-power mode, a low-power mode, and the like. Further, in the operation mode, a power-saving mode may be set in which the supply fan 34 and the exhaust fan 60 are operated with reduced power when the safety cabinet 40 is not in use. In the operation mode table 116, numerical values of the power for operating the supply fan 34 and the exhaust fan 60 are set for each operation mode.

The supply fan 34 and the exhaust fan 60 are operated while adjusting their power under the control of the control device 110. In addition, the actual power data of the supply fan 34 and the exhaust fan 60 are transmitted to the control device 110 and monitored by the calculation unit 114.

Note that, in the cleanroom system 10, by performing control to relatively increase the power of the supply fan 34, it is possible to maintain the cleanroom 20 at positive pressure (a state in which the pressure of the interior 22 is higher than the pressure of the exterior 90 on a time-average basis). The positive-pressure cleanroom 20 is suitable for applications in which it is desired to reduce or prevent fine particles from flowing into the interior 22 from the exterior 90. If there is a need for operation at positive pressure, the control device 110 may be configured to switch the cleanroom system 10 between negative pressure and positive pressure based on input from the UI 100.

In the above description, the supply fan 34 and the exhaust fan 60 are controlled while monitoring various pieces of data. However, the supply fan 34 and the exhaust fan 60 may operate without being controlled on the basis of the monitoring as long as the required negative pressure can be ensured.

In the above description, the supply and exhaust of air are performed from the ceiling portion. However, one or both of the supply and exhaust of air may be performed from a portion (wall or floor) other than the ceiling.

In the above description, the cleanroom system 10 uses the HEPA filters 32, 46, and 62 as filters for purifying air. HEPA is a high-precision particulate filter whose specific performance is defined by standards such as JIS, ISO, and EN. In the cleanroom system 10, the HEPA filters 32, 46, and 62 are installed according to appropriate standards. By using HEPA filters, it is expected that the required cleanliness can be ensured in many cases where the cleanroom system 10 is used. However, in the cleanroom system 10, some or all of the HEPA filters 32, 46, and 62 can be replaced with other filters according to the required level of cleanliness. For example, instead of HEPA filters, higher-performance filters (such as ULPA) or lower-performance filters may be used. Note that HEPA filters capture fine particles utilizing the fineness of the filter, but there are also types that utilize static electricity. In the type utilizing static electricity, in general, it is necessary to constantly generate static electricity by performing continuous operation in order to continuously adsorb fine particles. In the type utilizing static electricity, in a case where the above-described power-saving mode is implemented, for example, the air supply and exhaust volume may be controlled at a level at which the fall of fine particles can be prevented or limited to a predetermined level.

In the above description, the cleanroom system 10 is provided with the buffer room 24. However, the cleanroom system 10 may be formed compactly without providing the buffer room 24. In another embodiment, the buffer room 24 may be dedicated to entry, and a buffer room dedicated to exit may be separately provided.

In the above description, the safety cabinet 40 which is an open-type hazard control cabinet has been taken as an example. However, the above embodiment can be easily applied to other open-type hazard control cabinets, such as a draft chamber, in addition to the safety cabinet 40.

In a draft chamber, air drawn from the front opening (corresponding to a workspace air supply path) is directly supplied to the workspace. In addition, a workspace exhaust path for exhausting air from the workspace to the exterior of the cleanroom is provided, and an exhaust fan and a filter are installed in the workspace exhaust path. The flow path consisting of the front opening, the workspace, and the workspace exhaust path corresponds to the room exhaust path, and the workspace exhaust path coincides with the common exhaust path. In addition, by not providing an exhaust path leading to the exterior of the cleanroom, it is possible to form a negative-pressure cleanroom system in which the exhaust fan drives the total exhaust and the filter purifies the total exhaust.

### (2) Second Embodiment

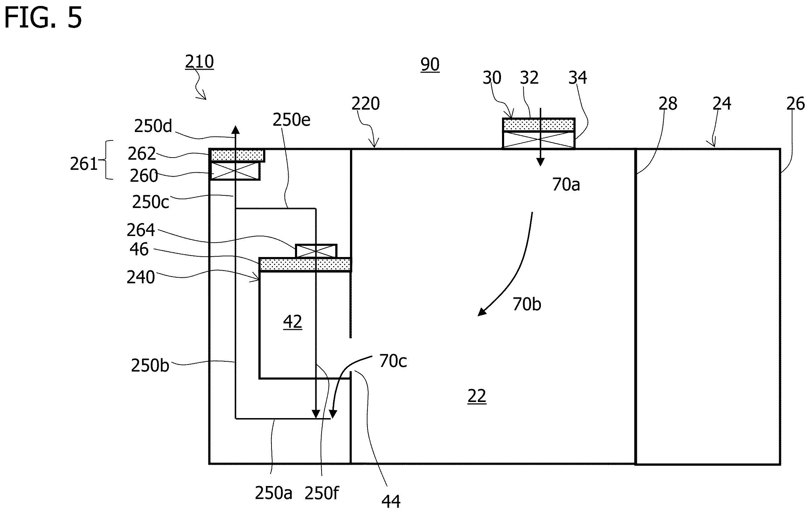

A cleanroom system 210 according to the second embodiment will be described with reference to FIG. 5. FIG. 5 is a side view according to the second embodiment. In FIG. 5, the same or substantially similar parts as those in FIG. 3 (the side view according to the first embodiment) are denoted by the same reference numerals, and the description thereof will be simplified or omitted.

In the cleanroom system 210, a cleanroom 220 includes an air supply unit 30 and a safety cabinet 240. The safety cabinet 240 includes a workspace 42, a front opening 44, a HEPA filter 46, flow paths 250a to 250f, an exhaust unit 261, and a workspace supply fan 264. The flow paths 250a to 250f are formed at substantially the same positions and with the same shapes as the flow paths 50a to 50f of the first embodiment, respectively.

However, the upper end of the flow path 250b communicates with the lower end of the flow path 250c and the rear end of the flow path 250e. The exhaust unit 261 is detachably attached near the connection point of the flow paths 250c and 250d. The exhaust unit 261 is a unit in which the exhaust fan 260 and the HEPA filter 262 disposed immediately above the exhaust fan are combined. The HEPA filter 262 is a filter that purifies exhaust air, and is detachably attached to the exhaust unit 261. Therefore, during cleaning or replacement of the HEPA filter 262, the exhaust unit 261 is first removed, and then the HEPA filter 262 is removed from the exhaust unit 261. The workspace supply fan 264 is provided near the front end of the flow path 250e. The workspace supply fan 264 is a fan that drives the supply of air to the workspace 42.

In the second embodiment, the workspace air supply path is a flow path that starts from the interior 22 and reaches the workspace 42 via the front opening 44, the lower front part of the workspace 42 (the lower front part of the flow path 250f), the flow path 250a, the flow path 250b, and the flow path 250e. The workspace exhaust path is a flow path that starts from the workspace 42 and reaches the exterior 90 via the flow paths 250a, 250b, 250c, and 250d. The room exhaust path is a flow path that starts from the interior 22 and reaches the exterior 90 via the front opening 44, the lower front part of the workspace 42, and the flow paths 250a, 250b, 250c, and 250d. The common exhaust path is a flow path consisting of the flow paths 250a, 250b, 250c, and 250d. The circulation path consists of the workspace 42 (the flow path 250f) and the flow paths 250a, 250b, and 250e.

The exhaust fan 260 drives the exhaust of air from the interior 22 to the exterior 90 through the front opening 44, the lower front part of the workspace, and the flow paths 250a, 250b, 250c, and 250d. On the other hand, the exhaust fan 260 does not drive the flow in the flow path 250e and the workspace 42. This can be understood by considering a state in which only the exhaust fan 260 is operated and the workspace supply fan 264 is stopped. The workspace supply fan 264 is a circulation drive fan that drives only the flow in the circulation path consisting of the workspace 42, the flow path 250a, the flow path 250b, and the flow path 250e. This can be understood by considering a state in which the exhaust fan 260 is stopped and only the workspace supply fan 264 is operated. Of course, in a case where the exhaust fan 260 and the workspace supply fan 264 are operated simultaneously, a nonlinear effect is generated in addition to the linear superposition. However, since this nonlinear effect is limited, if the effect of the workspace supply fan 264 on the exhaust is not considered, it can be considered that the exhaust fan 260 provided in the common exhaust path drives the total exhaust. In addition, it can be considered that the HEPA filter 262 provided in the common exhaust path purifies the total exhaust. In the second embodiment, there is redundancy that the workspace supply fan 264 is provided as compared to the first embodiment, but the exhaust structure is simply realized.

In the above description, an example has been described in which only one safety cabinet 240 is installed inside the cleanroom 220. However, in the cleanroom 220, a plurality of hazard control cabinets of the same or different types can be installed. In an aspect in which a plurality of hazard control cabinets is provided, it is necessary to provide a workspace exhaust path for exhausting air from each workspace to the exterior 90 of the cleanroom 220. The workspace exhaust paths may separately communicate with the exterior 90 without merging, and an exhaust fan and a filter may be provided in each of the exhaust paths. Further, the workspace exhaust paths may be merged so that the merging passage communicates with the exterior 90. In the case of merging, the exhaust fan and the filter may be provided only in the merging passage. In a case where the exhaust fan is provided only in the merging passage, a circulation drive fan may be provided in each of the hazard control cabinets to drive the circulation of air in each of the hazard control cabinets.

### (3) Third Embodiment

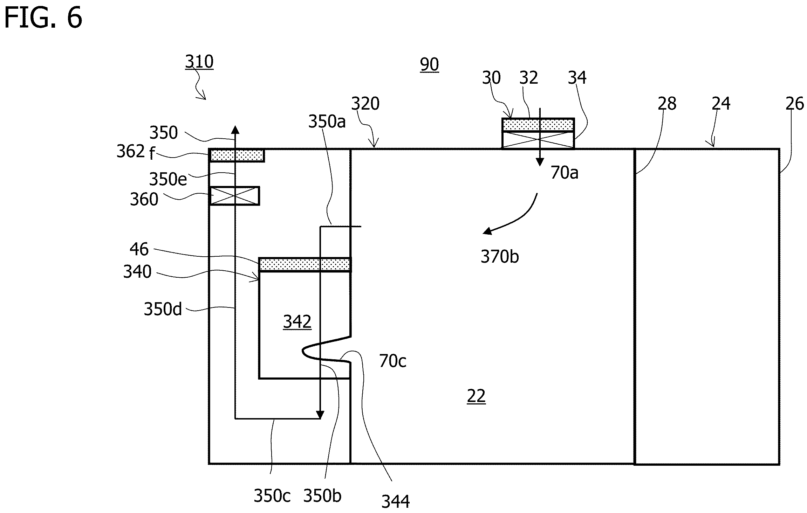

A cleanroom system 310 according to the third embodiment will be described with reference to FIG. 6. FIG. 6 is a side view according to the third embodiment. In FIG. 6, the same or substantially similar parts as those in FIG. 3 (the side view according to the first embodiment) are denoted by the same reference numerals, and the description thereof will be simplified or omitted.

In the cleanroom system 310, a cleanroom 320 includes an air supply unit 30 and an isolator 340. The isolator 340 is a closed-type hazard control cabinet, and includes a workspace 342 without any opening. A glove box 344 is provided on the front surface of the isolator 340 instead of a front opening. In the glove box 344, a glove having flexibility is arranged to protrude toward the workspace 342. The glove has airtightness, and an operator who inserts their hands into the glove can operate a work object inside the workspace 342 from the exterior of the workspace 342.

Flow paths 350a to 350f are provided around the workspace 342. The flow path 350a is a flow path for supplying air to the isolator 340 from the interior 22 of the cleanroom 320. The flow path 350a is located above the workspace 342 and extends substantially horizontally in the front-rear direction. The front end of the flow path 350a is open to the interior 22 of the cleanroom 320. The flow path 350b is a flow path for air flowing substantially uniformly downward through the workspace 342. The upper end of the flow path 350b communicates with the rear end of the flow path 350a via the HEPA filter 46. The HEPA filter 46 is a filter that purifies the air flowing into the workspace 342. A flow path 350c is a flow path provided below the workspace 342, and extends substantially horizontally in the front-rear direction of the isolator 340. The flow path 350c communicates with the flow path 350b via a hole provided in the bottom surface of the workspace 342. A flow path 350d is a flow path provided behind the workspace 342 and extends in the up-down direction, with its upper end reaching a position higher than the upper surface of the workspace 342. The lower end of the flow path 350d communicates with the rear end of the flow path 350c. A flow path 350e is a flow path provided on an extension line of the flow path 350d, and extends in the up-down direction. The lower end of the flow path 350e communicates with the upper end of the flow path 350d via an exhaust fan 360. The flow path 350f is a flow path provided on an extension line of the flow path 350e, and extends in the up-down direction. The lower end of the flow path 350f communicates with the upper end of the flow path 350e via a HEPA filter 362. The upper end of the flow path 350f is open to the exterior 90 of the cleanroom 320.

The exhaust fan 360 is a fan that drives exhaust air. The exhaust fan 360 is provided between the flow path 350d and the flow path 350e. The HEPA filter 362 is a filter that purifies exhaust air. The HEPA filter 362 is provided between the flow path 350e and the flow path 350f.

In the cleanroom system 310, air 70a is supplied to the interior 22 of the cleanroom 320 through the air supply unit 30. In the interior 22, air 370b flows in approximately one direction and reaches the front end of the flow path 350a. The air flowing through the flow path 350a is purified by the HEPA filter 46, then flows through the flow path 350b inside the workspace 342, and enters the flow path 350c through a hole provided in the bottom surface of the workspace 342. The air that has entered the flow path 350c moves to the flow path 350d, is then sucked by the exhaust fan 360, and is discharged to the flow path 350e. Then, the air is purified by the HEPA filter 362, passes through the flow path 350f, and then is exhausted to the exterior 90.

Since only the air purified by the HEPA filter 46 flows into the workspace 342 of the isolator 340, a high level of cleanliness can be achieved. In addition, since the air exhausted from the workspace 342 is purified by the HEPA filter 362, fine particles generated in the workspace 342 do not leak to the exterior 90. Of course, in the isolator 340, fine particles from the workspace 342 do not leak into the interior 22 of the cleanroom 320.

In the cleanroom system 310, the workspace air supply path corresponds to the flow path 350a, and the workspace exhaust path corresponds to the flow paths 350c, 350d, 350e, and 350f. The room exhaust path corresponds to the flow paths 350a, 350b, 350c, 350d, 350e, and 350f, and the common exhaust path coincides with the workspace exhaust path.

The exhaust fan 360 is provided in the common exhaust path. The exhaust fan drives the supply of air in the workspace air supply path and the exhaust of air in the workspace exhaust path and the room exhaust path. In the cleanroom system 310, no air is exhausted to the exterior 90, except for the exhaust from the flow path 350f. Therefore, the exhaust fan 360 drives the total exhaust of the cleanroom system 310. The HEPA filter 362 is provided in the common exhaust path to purify the total exhaust. Also in the third embodiment, a simple exhaust structure is realized.

### (4) Fourth Embodiment

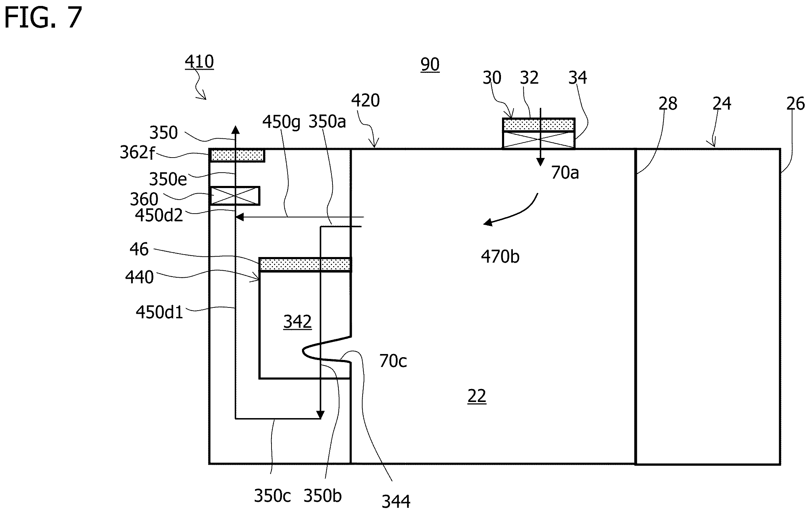

A cleanroom system 410 according to the fourth embodiment will be described with reference to FIG. 7. FIG. 7 is a side view according to the fourth embodiment. In FIG. 7, the same or substantially similar parts as those in FIG. 6 (the side view according to the third embodiment) are denoted by the same reference numerals, and the description thereof will be simplified or omitted.

In the cleanroom system 410, a cleanroom 420 includes an air supply unit 30 and an isolator 340. The isolator 440 is configured in substantially the same manner as the isolator in the third embodiment illustrated in FIG. 6.

The difference between the fourth embodiment and the third embodiment is the presence of a flow path 450g. The flow path 450g is a flow path located above the workspace 342, and extends substantially horizontally in the front-rear direction of the isolator 440. The front end of the flow path 450g is open to the interior 22 of the cleanroom 420. The rear end of the flow path 450g communicates with the upper end of a flow path 450d1 and the lower end of a flow path 450d2.

In the isolator 440, air flows similarly to the isolator 340 of the third embodiment. That is, air from the interior 22 of the cleanroom 420 is supplied to the workspace 342 through the flow path 350a. Then, air from the workspace 342 is exhausted to the exterior 90 through the flow paths 350c, 450d1, 450d2, 350e, and 350f. This exhaust is driven by the exhaust fan 360 and purified by the HEPA filter 362.

In addition, a flow is generated in which air from the interior 22 of the cleanroom 420 is exhausted to the exterior 90 via the flow paths 450g, 450d2, 350e, and 350f, without passing through the workspace 342. This is because the flow path 450g forms a bypass path that merges at the communicating point of the flow paths 450d1 and 450d2 without passing through the workspace 342. The exhaust of air via the flow path 450g is also driven by the exhaust fan 360 and purified by the HEPA filter 362. The exhaust volume passing through the flow path 450g can be adjusted by, for example, the cross-sectional area of the flow path 450g, or can be variably controlled by providing a movable valve in the flow path 450g.

In the fourth embodiment, the workspace air supply path corresponds to the flow path 350a, and the workspace exhaust path corresponds to the flow paths 350c, 450d1, 450d2, 350e, and 350f. There are two room exhaust paths: one corresponds to the flow path 350a, 350b, 350c, 450d1, 450d2, 350e, and 350f, and the other corresponds to the flow path 450g, 450d2, 350e, and 350f. The common exhaust path shared by all the room exhaust paths and the workspace exhaust path corresponds to the flow paths 450d2, 350e, and 350f. In addition, no other exhaust paths are provided. The exhaust fan 360 and the HEPA filter 362 are provided in the common exhaust path, and drive and purify the total exhaust, respectively.

### (5) Fifth Embodiment

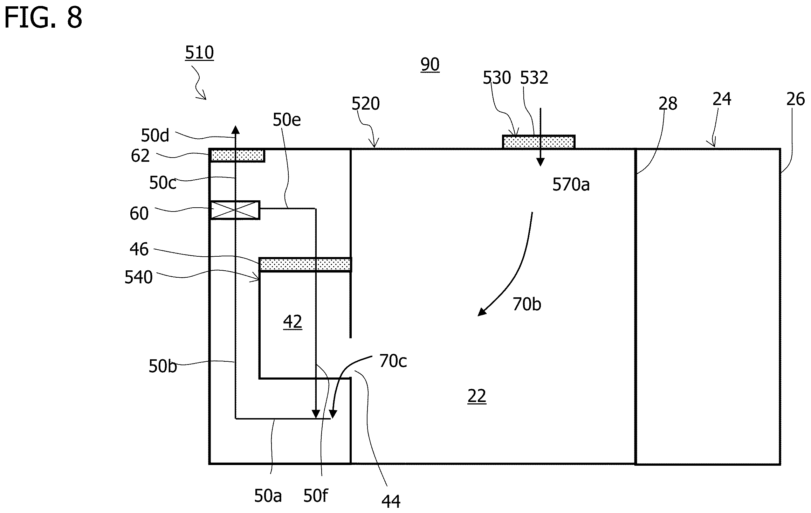

A cleanroom system 510 according to the fifth embodiment will be described with reference to FIG. 8. FIG. 8 is a side view according to the fifth embodiment. In FIG. 8, the same or substantially similar parts as those in FIG. 3 (the side view according to the first embodiment) are denoted by the same reference numerals, and the description thereof will be simplified or omitted.

In the cleanroom system 510 according to the fifth embodiment, unlike the cleanroom system 10 according to the first embodiment, the air supply unit 530 is not provided with a supply fan. The air supply unit 530 is provided with only the HEPA filter 532.

In the cleanroom system 510, the air volume in the interior 22 of the cleanroom 520 is reduced due to the exhaust of air by the exhaust fan 60, and negative pressure is created. The exhaust fan 60 operates with increased power to increase the degree of negative pressure in the interior 22. Therefore, the required amount of air 570 flows into the interior 22 from the exterior 90 by overcoming the air resistance in the HEPA filter 532 provided in the room air supply path. That is, the supply of air in the cleanroom system 510 is driven by a pressure difference between the interior 22 of the cleanroom 520 and the exterior 90. As far as the air supply portion is concerned, no power is used, and it can be called natural air supply.

In the fifth embodiment, the exhaust structure is simplified as in the first embodiment, and the room air supply path is a natural air supply path. Therefore, in the cleanroom system 510, there is a possibility that the manufacturing or maintenance costs can be reduced.

### (6) Sixth Embodiment

As described above, the negative-pressure cleanroom system has a biohazard control function that prevents or reduces the leakage of fine particles generated in the workspace. For this reason, the negative-pressure cleanroom system is often used when a substance having a negative effect on a human body or an environment is handled as a work object. Handling of a work object includes collection, testing, inspection, processing, manufacturing, and the like.

Examples of the work object for which biohazard control is required include radioactive substances. A radioactive pharmaceutical product is a pharmaceutical product that uses radioactive substances. In addition, chemical substances having toxicity or high physiological activity are also work objects for which hazard control is required. Anticancer drugs are examples of chemical substances having high physiological activity. Further, the biological objects are also work objects for which biohazard control is required. Here, the biological objects refer to some or all of organisms (including viruses) or objects derived from organisms that have been processed or otherwise treated. Examples of biological objects include tissues, cells, or nucleic acids of humans, animals, or microorganisms, or processed products thereof. Specimens such as blood are also examples of biological objects. Specimens related to highly transmissible infectious diseases that have a significant impact on public health require a high level of hazard control. In addition, antibody pharmaceutical products, nucleic acid pharmaceutical products, and regenerative medical products are also examples of biological objects. Biological objects require biohazard control because they may contain infectious or pathogenic substances, among other reasons.

In the sixth embodiment, as an example where biohazard control is required, a usage form of a cleanroom system in the regenerative medicine field related to autologous cell transplantation will be described.

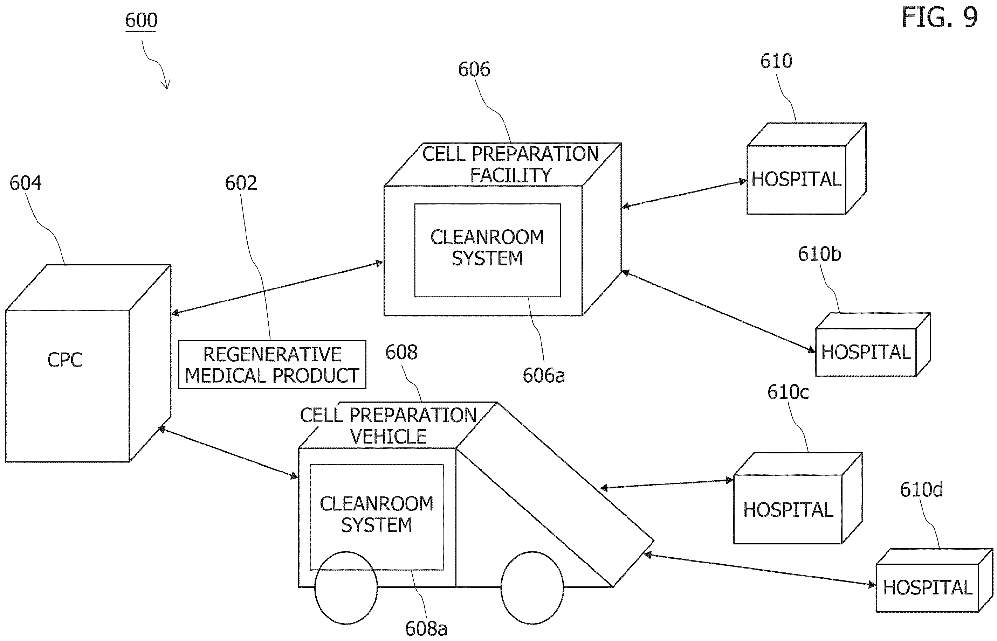

FIG. 9 is a diagram for explaining a network 600 according to the sixth embodiment. The network 600 illustrated in FIG. 9 connects transport-related facilities of a regenerative medical product 602. The network 600 includes a CPC 604, a cell preparation facility 606, a cell preparation vehicle 608, and hospitals 610a, 610b, 610c, and 610d (some or all of which may be referred to as a hospital 610).

The CPC 604 is a cell processing center (cell culture processing facility), and is sometimes referred to as a CPF (Cell Processing Facility). The CPC 604 includes a cleanroom and processes cells collected from a patient to manufacture the regenerative medical product 602. The cleanroom systems as exemplified in the first to fifth embodiments can be introduced into the CPC 604. However, the CPC 604 is a relatively large facility, and may include a plurality of cleanrooms having a large area, or may include a plurality of biohazard control cabinets in a cleanroom. Therefore, in the cleanroom of the CPC 604, it is also conceivable to provide an exhaust mechanism in the cleanroom itself in addition to the biohazard control cabinet.

The cell preparation facility 606 is a facility provided between the CPC 604 and the hospital 610. The cell preparation facility 606 performs cell preparation and transportation for the cells collected from patients or the manufactured regenerative medical product 602. The cell preparation facility 606 is installed inside a building, and includes a cleanroom system 606a as exemplified in the first to fifth embodiments. The cell preparation is performed using a workspace of a hazard control cabinet installed in the cleanroom system 606a. Note that the cell preparation broadly refers to a process that alters the state of cells or a process that tests or inspects the state of cells.

The cell preparation vehicle 608 is a vehicle used between the CPC 604 and the hospital 610 to prepare and transport cells collected from patients or manufactured regenerative medical products. The cell preparation vehicle 608 is formed by mounting the cleanroom system 608a as exemplified in the first to fifth embodiments on a self-propelled vehicle including an electric motor, an internal combustion engine, and the like.

The hospital 610 is a facility where a person performing regenerative medicine such as physicians, dentists, and veterinarians diagnoses and treats patients. The hospitals 610a to 610d are assumed to be established by different organizations and installed in buildings at different locations. In the hospital 610, regenerative medicine related to autologous transplantation is performed. Physicians and dentists are responsible for collecting tissue or cells from patients and transplanting regenerative medical products 602 into patients.

In the sixth embodiment, it is assumed that the number of installations of the hospitals 610 is the largest, the number of installations of the cell preparation facilities 606 or the cell preparation vehicles 608 is the second largest, and the number of installations of the CPCs 604 is the smallest. Then, the CPC 604 provides a service for a plurality of hospitals 610 via a plurality of cell preparation facilities 606 or cell preparation vehicles 608. In addition, the cell preparation facility 606 or the cell preparation vehicle 608 provides services to a plurality of hospitals 610. Of course, a hospital 610 may use a plurality of cell preparation facilities 606 or cell preparation vehicles 608, and a cell preparation facility 606 or cell preparation vehicle 608 may use a plurality of CPCs 604.

When regenerative medicine related to autologous transplantation is performed, first, cells are collected from one or a plurality of target patients at the hospital 610. During collection, the staff from the cell preparation facility 606 or the cell preparation vehicle 608 visit the hospital 610 to assist with the collection. Examples of the collection assistance include a process of inputting patient information, cell information, and the like to a computer, a process of storing the collected cells in a container whose temperature and cleanliness are controlled, and a process of adjusting the transplantation schedule.

The staff bring the collected cells back to the cell preparation facility 606 or the cell preparation vehicle 608. Then, necessary cell preparation is performed using the cleanroom systems 606a and 608a. Examples of cell preparation include one or more treatments selected from cell washing, drug addition, refrigeration, freezing, container storage, and infectious specimen testing including sterility tests.

The staff transport the cells contained in the container to the CPC 604. Transportation may be entrusted to a courier or the staff from the CPC 604, or the staff may transport the cells themselves. The transportation may be performed individually for each person, or may be performed collectively for a plurality of persons.

At the CPC 604, regenerative medical products 602 are manufactured from the received cells. In the manufacturing process, for example, cell culture is performed using manufacturing equipment installed in the cleanroom. The manufactured regenerative medical products 602 are transported to the cell preparation facility 606 or the cell preparation vehicle 608 in a frozen state.

In the cell preparation facility 606, the received regenerative medical products 602 are stored in a storage in which cleanliness and temperature are controlled. The storage may be provided in the cleanroom systems 606a and 608a or may be provided externally.

The regenerative medical products 602 are taken out of the storage at timing calculated backward from the scheduled transplantation date and time at the hospital 610. Then, cell preparation is performed in the cleanroom systems 606a and 608a. Examples of cell preparation include one or more treatments selected from thawing, warming, activation, drug addition, container storage, and testing including sterility tests.

The staff from the cell preparation facility 606 or the cell preparation vehicle 608 deliver the prepared regenerative medical products 602 to the hospital 610. The staff may also provide assistance during transplantation of the regenerative medical products 602 as necessary. Examples of assistance include verification of patient information, cell information, and the like registered in the computer against the patient and the regenerative medical product 602.

As described above, the staff from the cell preparation facility 606 and the cell preparation vehicle 608 visit a plurality of hospitals 610 to support regenerative medicine. Therefore, it is convenient for the cell preparation facility 606 to be located near two or more, three or more, four or more, or five or more customer hospitals 610. Here, the range of "near" can be defined by the one-way travel time between the patient's treatment location at the hospital 610 and the cleanroom system 606a at the cell preparation facility 606. Specifically, the travel time may be within 90 minutes, 60 minutes, 40 minutes, 30 minutes, 20 minutes, 10 minutes, or 5 minutes. The travel time is measured as the time required using commonly used transportation means selected from walking, bicycle, motorcycle, automobile, bus, train, and the like. In order to equalize variations due to waiting times at signals, public transport intervals, and the like, the travel time is measured based on the average required time over multiple trips conducted on the same weekday and time of transportation. The range of "near" may also be defined based on the travel distance or straight-line distance using above-mentioned transportation means between the patient's treatment location at the hospital 610 and the cleanroom system 606a at the cell preparation facility 606. Specifically, the range of the travel distance or straight-line distance may be exemplified as within 20 km, 15 km, 10 km, 8 km, 6 km, 2 km, 1 km, or even 500 meters.

The cell preparation vehicle 608 can travel to the hospital 610 under its own power. However, the size of the cleanroom system 608a that can be mounted in the cell preparation vehicle 608 is limited. In a case where the mounting space of the vehicle is large, a buffer room may be provided in the cleanroom system 608a, but in order to save space, only the cleanroom may be provided without a buffer room. The cleanroom system 608a is designed to be usable even when the vehicle is traveling although it is generally used when the cell preparation vehicle 608 is stopped.

The cleanroom systems 606a and 608a included in the cell preparation facility 606 and the cell preparation vehicle 608 include the above-described simple exhaust mechanism. Therefore, the manufacturing or maintenance costs required for the exhaust mechanism can be reduced as compared to conventional cleanrooms installed in the CPC 604 or the like. Since the cell preparation facility 606 and the cell preparation vehicle 608 need to be installed in greater numbers than the CPC 604, it is of high value to introduce the low-cost cleanroom systems 606a and 608a.

In the above description, the regenerative medical products 602 for autologous transplantation are used as an example. The network configuration in which regenerative medical products are provided from the CPC 604 to the hospital 610 via the cell preparation facility 606 or the cell preparation vehicle 608 is also widely applicable to regenerative medical products for allogeneic transplantation. Regenerative medical products for allogeneic transplantation are manufactured at the CPC using cells or the like derived from sources other than the transplant patient and are stored in storage facilities as necessary.

In the above description, the cell preparation facility 606 and the cell preparation vehicle 608 are described as facilities that support a plurality of hospitals 610. Alternatively, the cell preparation facility 606 or the cell preparation vehicle 608 may be a facility dedicated to each individual hospital 610. In the case of dedicated facilities, it is conceivable that the cell preparation facility 606 may be located even closer to the hospital 610 than in the above example. Specifically, as the range of "near", locations where the travel time from the cell preparation facility 606 to the treatment location is within 10 minutes, 5 minutes, 3 minutes, or even 1 minute can be exemplified. Also, as examples of the nearby locations, the cell preparation facility 606 may be located on the same site as the treatment location, in the same building, or on the same floor level within the same building. In a case where the cell preparation facility 606 or the cell preparation vehicle 608 is provided for each individual hospital 610, since the number of cleanroom systems 606a and 608a to be installed will also increase, there is a significant advantage in adopting an exhaust structure that can be installed or maintained at low cost.

### REFERENCE SIGNS LIST

- 10: CLEANROOM SYSTEM
- 20: CLEANROOM
- 22: INSIDE
- 24: BUFFER ROOM
- 26: OUTER DOOR
- 28: INNER DOOR
- 30: AIR SUPPLY UNIT
- 32: HEPA FILTER
- 34: SUPPLY FAN
- 40: SAFETY CABINET
- 42: WORKSPACE
- 44: FRONT OPENING
- 46: HEPA FILTER
- 50a, 50b, 50c, 50d, 50e, 50f: FLOW PATH
- 60: EXHAUST FAN
- 62: HEPA FILTER
- 70a, 70b, 70c: Air
- 90: EXTERIOR
- 100: UI
- 102: OUTDOOR PRESSURE SENSOR
- 104: INDOOR PRESSURE SENSOR
- 106: INDOOR TEMPERATURE SENSOR
- 107: INDOOR PARTICLE SENSOR
- 108: WORKSPACE PARTICLE SENSOR
- 110: CONTROL DEVICE
- 112: RECORDING UNIT
- 114: ARITHMETIC UNIT
- 116: OPERATION MODE TABLE
- 210: CLEANROOM SYSTEM
- 220: CLEANROOM
- 240: SAFETY CABINET
- 250a, 250b, 250c, 250d, 250e, 250f: FLOW PATH
- 260: EXHAUST FAN
- 261: EXHAUST UNIT
- 262: HEPA FILTER
- 264: WORKSPACE SUPPLY FAN
- 310: CLEANROOM SYSTEM
- 320: CLEANROOM
- 340: ISOLATOR
- 342: WORKSPACE
- 344: GLOVE BOX
- 350a, 350b, 350c, 350d, 350e, 350f: FLOW PATH
- 360: EXHAUST FAN
- 362: HEPA FILTER
- 370b: AIR
- 410: CLEANROOM SYSTEM
- 420: CLEANROOM
- 440: ISOLATOR
- 450d1, 450d2, 450g: FLOW PATH
- 470b: AIR
- 510: CLEANROOM SYSTEM
- 520: CLEANROOM
- 530: AIR SUPPLY UNIT
- 532: HEPA FILTER
- 570: AIR
- 600: NETWORK
- 602: REGENERATIVE MEDICAL PRODUCT
- 604: CPC
- 606: CELL PREPARATION FACILITY
- 606a: CLEANROOM SYSTEM
- 608: CELL PREPARATION VEHICLE
- 608a: CLEANROOM SYSTEM
- 610, 610a, 610b, 610c, 610d: HOSPITAL

## Claims

1. A cleanroom system comprising:
a cleanroom having airtightness;
a room air supply path that supplies air from an exterior to an interior of the cleanroom; and
a hazard control cabinet installed inside the cleanroom, the hazard control cabinet including: a workspace; a workspace air supply path that supplies air from the interior of the cleanroom to the workspace; a circulation path that takes in air from the workspace and supplies the air to the workspace again; a workspace exhaust path that exhausts air from the workspace to the exterior of the cleanroom; an exhaust fan that drives circulation in the circulation path and exhaust in the exhaust path; and a filter that is provided in the workspace exhaust path and purifies the exhaust, wherein
the exhaust fan that drives circulation in the circulation path and exhaust in the exhaust path drives total exhaust from the interior of the cleanroom to the exterior, and maintains the interior of the cleanroom at negative pressure.

2. The cleanroom system according to claim 1, comprising:
a bypass path that communicates with the workspace exhaust path from the interior of the cleanroom without passing through the workspace, wherein
the exhaust fan also drives air flowing through the bypass path.

3. The cleanroom system according to claim 1, wherein
the hazard control cabinet is a safety cabinet, and
the workspace is a space in which work is performed on biological objects.

4. The cleanroom system according to claim 1, wherein
the room air supply path is a natural air supply path that supplies air using negative pressure inside the cleanroom formed by the exhaust fan as a driving force.

5. The cleanroom system according to claim 1, wherein
the workspace is a space in which work is performed on regenerative medical products manufactured in a cell culture processing facility, and
the cleanroom system is installed near treatment locations where the regenerative medical products are used.

6. The cleanroom system according to claim 5, wherein
the treatment locations are distributed in two or more buildings, and
the cleanroom system is installed at a position near the treatment locations in at least two or more buildings.

7. A vehicle mounted with a cleanroom system, comprising:
the cleanroom system according to claim 1; and
a vehicle on which the cleanroom is mounted, wherein
the workspace is a space in which work is performed on regenerative medical products manufactured in a cell culture processing facility, and
the vehicle is capable of moving near treatment locations where regenerative medical products are used.
